(19) Europäisches Patentamt
European Patent Office
Office européen des brevets

(11) Publication number: 0 006 340
B1

# (12) EUROPEAN PATENT SPECIFICATION

(45) Date of publication of patent specification: 06.10.82

(51) Int. Cl.³: A 01 N 59/12, A 61 K 33/18

(21) Application number: 79301112.3

(22) Date of filing: 12.06.79

## (54) Method for the production of iodophor powders.

(30) Priority: 12.06.78 US 914683

(43) Date of publication of application:
09.01.80 Bulletin 80/1

(45) Publication of the grant of the patent:
06.10.82 Bulletin 82/40

(84) Designated Contracting States:
AT BE CH DE FR GB IT LU NL SE

(56) References cited:
FR - A - 2 293 441
FR - A - 2 293 458
FR - A - 2 351 663

(73) Proprietor: Mundipharma A.G.
St. Alban-Vorstadt 94 Postfach
CH-4006 Basel (CH)

(72) Inventor: Hofer, Peter
Birmanstrasse 9
CH-4410 Liestal (CH)

(74) Representative: Smith, Sydney (GB) et al,
Elkington & Fife 52/54 High Holborn
GB-London WC1V 6SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 006 340 B1

# Method for the production of iodophor powders

This invention relates to a method of producing an iodophor powder.

Iodophor compounds are well known germicidal agents comprising the combination of iodine with an organic carrier, particularly povidone, as well as cationic and anionic and nonionic detergents. In U.S. Patent No. 2,706,701 and in U.S. Patent No. 2,739,922 processes were disclosed for the production of polyvinylpyrrolidone iodine (PVP-I) by either dry mixing of the PVP and the iodine at 90—100°C, or by preparing solutions of both resulting in an iodophor with reduced iodine toxicity and without iodine vapour. It was claimed in these patents that stability is reached after the formation of an iodine to iodide ratio of 2:1.

In U.S. Patent No. 2,900,305 an improvement in the dry mixing process is described in which PVP with about 10% of water was used. In U.S. Patent No. 4,017,407 and U.S. Patent No. 4,038,476 the use of PVP-iodide in place of PVP for faster dissolution of the iodine in PVP solution or in dry mixing was described.

French Patent Specification No. 2,293,441 describes a process for the preparation of complexes of poly - N - vinylpyrrolidone (PVP) and iodine, in which process, there are prepared

(a) a solution and/or colloidal suspension of elemental iodine and a substance supplying iodide ions in first solvent or solvent mixture and

(b) a solution and/or colloidal solution and/or colloidal suspension of PVP in a second solvent or solvent mixture which has a surface tension which is different from that of the first solvent or solvent mixture, in which second solvent PVP is at least partially soluble or wettable, and in which the substances in solution or in suspension in the first solvent or solvent mixture are insoluble or sparingly soluble;

the solutions or suspensions a) and b) are combined with uniform mixing, in small portions, forming a multiphase system, mixing of the multiphase system is continued, the agglomerates formed which contain the iodine-PVP complex and the iodide ions are separated, and the products are dried.

French Patent No. 2,351,663 relates to complexes of molecular iodine with an insoluble polymeric organic matrix, for example polyvinyl pyrrolidone, and to their preparation and use in a number of applications in which the oxidative action of elementary iodine is required. The complexes are prepared by heating together an aqueous suspension of the organic polymer with a solution of iodine in a hydrocarbon solvent. There is no indication in the French specification that the complexes obtained are further treated by reaction with H1 and it is stressed at various points in the specification that the iodine in present in molecular form only.

The present invention provides a method for the production of an iodophor powder, characterised in that an organic iodophor-forming compound or compounds is suspended in a solution of iodine in a solvent in which the iodophor-forming compound is insoluble, to form a complex between the iodophor-forming compound and the iodine after which a solution of an iodide in a solvent system miscible with the solvent of the iodine solution is slowly added, preferably until the iodine solution becomes colourless. This causes the iodophor-forming compound to become bound to the iodine, thus forming a homogeneous iodophor powder.

For completion of double bond iodination, the powder may be heated up to 100°C until solutions in water show constant amount of available iodine, preferably 0.5—1%. The percentage of available iodine in the powder may be up to 25% the preferred percentage being from 5—20%.

The use of iodine in solution greatly increases the rate of complexing with the iodophor-forming compound while at the same time, the use of solid iodophor avoids the need for an extra drying step, e.g. spray drying, and still further, the initial addition of appropriate amounts of iodide prevents the formation of iodine vapours from starting, so that the overall process is a simple, clean reaction compared to the previous processes. Still further, the initial solvent for the iodine can be recycled after washing with water.

The process of the present invention is applicable to the use of all organic iodophor-forming compounds which include the various surfactant compounds such as poloxalenes as well as various non-surfactant compounds such as polyvinylpyrrolidone.

Polyvinylpyrrolidone, which is also abbreviated to PVP or "povidone" is generally considered to be the most useful of the iodophor-forming compounds, and the method of this invention is particularly applicable thereto. However, the method of the present invention may also be applied to the preparation of other iodophor complexes which comprise the non-ionic, cationic and anionic detergent carriers.

The non-ionic surface active agents include, for example the non-ionic polyglycol ether type surface active agents which are obtained by condensing alkylene oxides with water-insoluble organic compounds containing at least six carbon atoms and having an active hydrogen, such as organic hydroxy compounds, i.e., alcohols, phenols, thiophene, primary and secondary amines, carboxylic and sulphonic acids and their amides. Non-ionic polyglycol ether type surface active agents of this class are well known in the art and are disclosed, together with methods for their preparation in U.S. Patents Nos. 1,970,578 and 2,213,477.

These agents may be represented by the general formula:

$$R—(—CHR'—CHR'—O—)_n—H$$

in which R represents the residue of an organic compound containing an active hydrogen and R' represents hydrogen or lower alkyl and 'n' represents an integer of from 3 to 100 or higher, preferably from 6 to 50. These compounds are readily obtained by the methods disclosed in the above mentioned patents by condensing a polyglycol ether containing the required number of alkyleneoxy groups, or an alkylene oxide, usually ethylene oxide, propylene oxide or butylene oxide, with a water-insoluble organic compound containing at least six carbon atoms and having an active hydrogen for example an alkylphenol.

Other members of the group of non-ionic surfactants also may be used in this new process to prepare iodophors for example, the class of non-ionic surfactants characterized by the condensation of polyoxypropyleneglycol with ethylene oxide containing various chain lengths. Such non-ionic agents are disclosed and claimed in U.S. Patent 2,647,619 and have the general formula:

$$HO—(C_2H_4O)_z\,(C_3H_6)_y\,(C_2H_4O)_{z'}H$$

in which y equals at least 15; and ($C_2H_4$) z+z' represents 20 to 90 percent of the total weight of the compound. These non-ionic surface active agents are available commercially and are known by the trade name Pluronics, a product of Wyandotte Chemicals Corporation of Wyandotte, Michigan.

Among the suitable anionic surface active agents which can be used for the formation of iodophors in accordance with the present invention are those represented by the formula:

$$\begin{array}{l} R—N—CH_2—CH_2—SO_3—Y \\ \quad\;\; | \\ \quad\;\; R' \end{array}$$

in which R is the radical $C_xH_{(2x+1)}CO$ CO; x being an integer of from 5 to 17 and R' represents hydrogen, or a ($C_1$—$C_4$) alkyl or cyclohexyl radical and Y represents a salt-forming cation. The preferred anionic detergent compounds are of the well known groups of anionic surface active agents known as alkanoyl taurates and alkylaryl sulphonates such as alkyl benzene sodium sulphonate and alkyl naphthyl sodium sulphonate.

Among the suitable cationic surface active agents that may be used as iodophor-forming compounds in accordance with the invention are quaternary ammonium salts such as those formed by the alkylation of fatty amines; straight chain fatty amine salts having from 8 to 18 carbon atoms in chain length, as for example, octadecyl amine; amino amides and imidazolines.

In general, the present invention will be discussed with respect to the use of PVP as the iodophor-forming compound. It is to be understood, however, that any of the other solid organic iodophor-forming compounds can be used, and in fact mixtures can be used. The most suitable solvents for the iodine or mixed halogen, in which the organic iodophor-forming compound, particularly PVP, is insoluble, are the saturated alkanes or halogenated alkanes, preferably with boiling points of at least 45°C.

The iodide which is added in the form of a solution in a solvent system miscible with the solvent for the iodophor-forming compound is preferably in the form of hydrogen iodide or ammonium iodide. However, any other source of iodide ion can be used, for example, potassium iodide, sodium iodide or lithium iodide.

The most suitable solvent system for the iodide, that is a solvent system in which the iodide is soluble and which is miscible with the solvent for the iodide in which the iodophor-forming compound is insoluble, is a mixture of a small amount of water with a larger amount of an alkanol or alkanone. The most suitable alkanols are methanol, ethanol and propanol. Other aliphatic alcohols can be used. The preferred aliphatic ketones are acetone and 2-butanone.

The solution of the iodide in the solvent system miscible with the solvent for the iodide is added slowly to the suspension of the iodophor-former in the iodine solution, until the iodine solution becomes colourless. This generally requires about two hours. In the case of the use of PVP as the iodophor-former, simple filtration results in a homogeneous PVP-I powder being obtained. The use of a mixed polymer iodophor-former, such as PVP and dextran or polymerised dextran up to a ratio of 3 to 5 in case of 10% available iodine results in the formation of the corresponding mixed polymer iodophor powder. When using PVP, it is desirable to use a good quality PVP (K15 to K90). As indicated above, double bond iodination is completed by heating of the powder to 100°C until solutions in water show constant amounts of available iodine.

The invention will now be further described with reference to the following Examples.

Example 1

8.5 g of PVP (K30) were suspended in a solution of 1.1 g of iodine in 200 ml of petroleum ether. A solution of 0.363 ml of 56% HI in 5 ml of acetone was added slowly. After 2 hr the solvent was colourless and the PVP-I was collected on a filter (10.7 g). Titration with 0.01 N $Na_2S_2O_3$ solution gave 10.0% of available iodine.

Example 2

The amount of PVP (K30) was reduced to 3.3 g and processed according to Example 1.

Collection gave 5.1 g of PVP-I with 20.2% of available iodine.

Example 3

6.6 g of PVP (K17) were suspended in a solution of 2.2 g of iodine in 400 ml of petroleum ether. A solution of 0.528 ml of 67% HI in 10 ml of acetone was added slowly. Collection after 2 hr gave 10.0 g of PVP-I with 19.8% of available iodine.

Example 4

2.7 g of PVP (K30) were suspended in a solution of 1.1 g of iodine in 200 ml of petroleum ether. A solution of 0.4 g of $NH_4I$ in 0.2 ml of water and 5 ml of acetone was added slowly. Collection after 3 hr gave 4.4 g of PVP-I with 20.2% of available iodine.

Example 5

2.3 g of PVP (K30) were suspended in a solution of 1.1 g of iodine in 200 ml of petroleum ether. A solution of 0.264 ml of 67% HI in 5 ml of acetone was added slowly. Collection after 3 hr gave 4.1 g of PVP-I, with 24.6% of available iodine.

Example 6

8.5 g of PVP (K30) were suspended in a solution of 1.41 g of iodine monochloride in 200 ml of petroleum ether. A solution of 0.264 ml of 67% HI in 5 ml of acetone was added slowly. Collection after 3 hr gave 10.2 g of PVP-ICl with 8.6% of titratable iodine.

**Claims**

1. A method of producing an iodophor powder, characterised in that an organic iodophor-forming compound is first suspended in a solution of iodine dissolved in a solvent in which the iodophor-forming compound is insoluble and an iodide dissolved in a solvent system miscible with the solvent for the iodine, is then added.

2. A method as claimed in claim 1 characterised in that the iodine solution is added until the iodine solution becomes colourless.

3. A method as claimed in claim 1 or claim 2 characterised in that the iodophor-forming compound is polyvinylpyrrolidone.

4. A method as claimed in any of claims 1 to 3 characterised in that the solvent for the iodine is a saturated alkane or a halogenated alkane.

5. A method as claimed in any of claims 1 to 4 characterised in that the solvent system for the iodide comprises water and an aliphatic alcohol or ketone.

6. A method as claimed in claim 5 characterised in that the amount of water in the solvent system is less than the amount of aliphatic alcohol or ketone.

7. A method as claimed in any of claims 1 to 6 characterised in that the iodide is hydroiodic acid, ammonium iodide, lithium iodide, sodium iodide or potassium iodide.

8. A method as claimed in any of claims 1 to 7 characterised in that the amount of iodine relative to the amount of iodophor-forming compound is such that the resulting iodophor contains from 1 to 25% of available iodine.

9. A method as claimed in any of claims 1 to 8 characterised in that the iodophor-forming compound is a mixture of polyvinylpyrrolidone and dextran or polymerised dextran.

10. A method as claimed in claim 9 characterised in that the ratio of polyvinylpyrrolidone to dextran or polymerised dextran is up to a ratio of 3:5 in the case of 10% of available iodine.

**Revendications**

1. Procédé de préparation d'un iodophore en poudre, caractérisé en ce que l'on prépare en premier lieu une suspension d'une composé organique capable de former un iodophore dans une solution d'iode dissous dans un solvant, dans lequel le composé formant l'iodophore est insoluble, puis on y ajoute un iodure dissous dans un mélange solvant miscible avec le solvant pour l'iode.

2. Procédé suivant la revendication 1, caractérisé en ce que la solution d'iodure est ajoutée jusqu'au moment où la solution d'iode est devenue incolore.

3. Procédé suivant la revendication 1 ou la revendication 2, caractérisé en ce que le composé formant l'iodophore est la polyvinylpyrrolidone.

4. Procédé suivant l'une quelconque des revendications 1 à 3, caractérisé en ce que le solvant pour l'iode est un alcane saturé ou un alcane halogéne.

5. Procédé suivant l'une quelconque des revendications 1 à 4, caractérisé en ce que le mélange solvant pour l'iodure est composé d'eau et d'une cétone ou d'un alcool aliphatique.

6. Procédé suivant la revendication 5, caractérisé en ce que la proportion de l'eau dans le mélange solvant est inférieure à la proportion de la cétone ou de l'alcool aliphatique.

7. Procédé suivant l'une quelconque des revendications 1 à 6, caractérisé en ce que l'iodure est choisi parmi l'acide iodhydrique, l'iodure d'ammonium, l'iodure de lithium, l'iodure de sodium et l'iodure de potassium.

8. Procédé suivant l'une quelconque des revendications 1 à 7, caractérisé en ce que les proportions relatives de l'iode et du composé formant l'iodophore sont telles que l'iodophore formé contient entre 1 et 25% d'iode disponible.

9. Procédé suivant l'une quelconque des revendications 1 à 8, caractérisé en ce que le composé formant l'iodophore est un mélange de polyvinyl-pyrrolidone et de dextrane ou de dextrane polymérisé.

10. Procédé suivant la revendication 9, caractérisé en ce que le rapport entre la polyvinyl-pyrrolidone et le dextrane ou le dextrane polymérisé est de 3:5 dans un iodophore à 10% d'iode disponible.

**Patentansprüche**

1. Verfahren zur Herstellung eines Jodophor-Pulvers, dadurch gekennzeichnet, daß man eine organische, einen Jodophor bildende Verbindung zunächst in einer Lösung von Jod in einem Lösungsmittel, in dem die den Jodophor bildende Verbindung unlöslich ist, suspendiert und daß man sodann ein Jodid zusetzt, welches in einem Lösungsmittel-system aufgelöst ist, das mit dem Lösungsmittel für das Jod mischbar ist.

2. Verfahren nach Anspruch 1, dadurch gekennzeichnet, daß man die Jodidlösung so lange zusetzt, bis die Jodlösung farblos wird.

3. Verfahren nach Anspruch 1 oder 2, dadurch gekennzeichnet, daß die den Jodophor bildende Verbindung Polyvinylpyrrolidon ist.

4. Verfahren nach einem der Ansprüche 1 bis 3, dadurch gekennzeichnet, daß das Lösungsmittel für das Jod ein gesättigtes Alkan oder ein halogeniertes Alkan ist.

5. Verfahren nach einem der Ansprüche 1 bis 4, dadurch gekennzeichnet, daß das Lösungsmittelsystem für das Jodid Wasser und einem aliphatischen Alkohol oder ein Keton umfaßt.

6. Verfahren nach Anspruch 5, dadurch gekennzeichnet, daß die Wassermenge in dem Lösungsmittelsystem geringer ist als die Menge des aliphatischen Alkohols oder des Ketons.

7. Verfahren nach einem der Ansprüche 1 bis 6, dadurch gekennzeichnet, daß das Jodid Jodwasserstoffsäure, Ammoniumjodid, Lithiumjodid, Natriumjodid oder Kaliumjodid ist.

8. Verfahren nach einem der Ansprüche 1 bis 7, dadurch gekennzeichnet, daß die Jodmenge relativ zu der Menge der den Jodophor bildenden Verbindung so bemessen ist, daß der resultierende Jodophor 1 bis 25% verfügbares Jod enthält.

9. Verfahren nach einem der Ansprüche 1 bis 8, dadurch gekennzeichnet, daß die den Jodophor bildende Verbindung ein Gemisch aus Polyvinylpyrrolidon und Dextran oder polymerisiertem Dextran ist.

10. Verfahren nach Anspruch 9, dadurch gekennzeichnet, daß das Verhältnis von Polyvinylpyrrolidon zu Dextran oder polymerisiertem Dextran bis zu einem Verhältnis von 3:5 im Falle von 10% verfügbarem Jod beträgt.